# EUROPEAN PATENT SPECIFICATION

(11) **EP 2 830 499 B1**
(45) Date of publication and mention of the grant of the patent: **20.02.2019**
(21) Application number: 13768938.6
(22) Date of filing: 29.03.2013
(51) Int. Cl.: A61B 5/157, A61M 5/142, A61M 5/158

(54) **FLUID DELIVERY DEVICE WITH TRANSCUTANEOUS ACCESS TOOL, INSERTION MECHANSIM AND BLOOD GLUCOSE MONITORING FOR USE THEREWITH**
FLÜSSIGKEITSABGABEVORRICHTUNG MIT EINEM INSTRUMENT FÜR TRANSKUTANEN ZUGANG UND EINEM EINSATZMECHANISMUS SOWIE BLUTZUCKERÜBERWACHUNGSVORRICHTUNG ZUR VERWENDUNG DAMIT
DISPOSITIF D'ADMINISTRATION DE FLUIDE AVEC OUTIL D'ACCÈS TRANSCUTANÉ, MÉCANISME D'INSERTION ET CONTRÔLE DE GLYCÉMIE DESTINE À ÊTRE UTILISÉ AVEC LE DISPOSITIF

(30) Priority: 30.03.2012 US 201261618028 P
(43) Date of publication of application: 04.02.2015
(73) Proprietor: Insulet Corporation, Billerica, MA 01821 (US)
(72) Inventor: DIIANNI, Steven, Groveland, Massachusetts 01834 (US); MCLAUGHLIN, Ian, Boxborough, MA 01719 (US); O'CONNOR, Jason B., Acton, Massachusetts 01720 (US); CAMPBELL, Robert, Waltham, Massachusetts 02453 (US); SCHMID, Kevin, Boxford, Massachusetts 01921 (US)
(74) Representative: CMS Cameron McKenna Nabarro Olswang LLP
(86) International application number: PCT/US2013/034674
(87) International publication number: WO 2013/149186

(56) References cited:
- EP-A1- 2 397 181
- WO-A1-98/56293
- WO-A1-2008/133702
- US-A1- 2002 032 374
- US-A1- 2003 163 097
- US-A1- 2006 178 633
- US-A1- 2006 253 085
- US-A1- 2007 282 269
- US-A1- 2008 004 515
- US-A1- 2008 004 515
- US-A1- 2009 062 767
- US-A1- 2009 062 767
- US-A1- 2011 054 399
- US-A1- 2011 230 833
- US-A1- 2012 078 161

## Description

### TECHNICAL FIELD

The present invention relates to fluid delivery devices for delivering therapeutic liquids to a patient, and more particularly, to an infusion pump for delivering therapeutic liquids to a patient.

### BACKGROUND INFORMATION

Fluid delivery devices have numerous uses such as delivering a liquid medicine or other therapeutic fluid to a patient subcutaneously. In a patient with diabetes mellitus, for example, ambulatory infusion pumps have been used to deliver insulin to a patient. These ambulatory infusion pumps have the ability to offer sophisticated fluid delivery profiles including variable basal rates and bolus requirements. The ability to carefully control drug delivery can result in better efficacy of the drug and therapy and less toxicity to the patient.

Some existing ambulatory infusion pumps include a reservoir to contain the liquid medicine and use electromechanical pumping or metering technology to deliver the liquid medicine via tubing to a needle and/or soft cannula that is inserted subcutaneously into the patient. These existing devices allow control and programming via electromechanical buttons or switches located on the housing of the device. The devices include visual feedback via text or graphic screens and may include alert or warning lights and audio or vibration signals and alarms. Such devices are typically worn in a harness or pocket or strapped to the body of the patient.

Some infusion pumps have been designed to be relatively small, low cost, light-weight, and easy-to-use. One example of such a pump is the OMNIPOD® insulin infusion pump available from Insulet Corporation. Examples of infusion pumps are also described in greater detail, for example, in U.S. Patent Nos. 7,128,727; 7,018,360; and 7,144,384 and U.S. Patent Application Publication Nos. 2007/0118405, 2006/0282290, 2005/0238507, and 2004/0010207, which are fully incorporated herein by reference. These pumps include insertion mechanisms for causing a transcutaneous access tool, such as a needle and/or soft cannula, to be inserted into a patient. Although such pumps are effective and provide significant advantages over other insulin infusion pumps, the design of the insertion mechanism may be improved, for example, to reduce the size of the pump, to improve the comfort to the user, and/or to incorporate continuous glucose monitoring (CGM). These pumps also include fluid driving mechanisms for driving fluid from a reservoir through the transcutaneous access tool. The fluid driving mechanisms may also be improved to facilitate assembly and use of the pump.

US 2006/253085 (Geismar, Eric P. et al.) provides a dual insertion set to include a base, an infusion portion, a sensor portion, and at least one piercing member. The base is adapted to secure the dual insertion set to the skin of a patient. The infusion portion includes a cannula for supplying a fluid to a placement site. The cannula is coupled to and extends from the base and has at least one lumen with a distal end for fluid communication with the placement site.

US 2008/004515 (Jennewine, R. Curtis) provides a method and system for providing an integrated analyte monitoring system and on-body patch pump with multiple cannulas and a sensor combination.

US 2011/054399 (Chong, Colin A. et al.) provides a first device housing configured to be operatively engaged with and disengaged from a base carried by a user and may include a first carrier body arranged for movement at least between a refracted position and an advanced position.

US 2002/032374 A1 (Holker, James D. et al.) provides a sensor and method of making the same for implantation in a body that includes a substrate with notches cut in the substrate to form a necked down region in the substrate; and at least one sensor electrode formed from one or more conductive layers. Preferably, the thickness of the substrate ranges from approximately 25 mu to 350 mu, but the thickness of the substrate can range from 5 mu to 750 mu.

EP 2397181 A1 (F. Hoffmann-La Roche AG) provides a device for inserting an infusion cannula or a sensor into a body of a patient comprises an insertion needle adapted to be removably arranged in the infusion cannula or sensor, a handle structure connected to the insertion needle,

US 2009/062767 A1 (Van Antwerp, Nannette M. et al.) provides a dual insertion set for supplying a fluid to the body of a patient and for monitoring a body characteristic of the patient.

US 2007/282269 A1 (Carter, Brett J. et al.) provides a disposable wearable infusion device having a body arranged to be adhered to a patient's skin and a reservoir for holding a liquid medicant to be infused into the patient. The infusion system further includes a cannula driver arranged to be detachably joined with the infusion device. The cannula driver includes a cannula and is arranged to drive the cannula into a deployed position extending from the infusion device to beneath the patient's skin.

WO98/56293 A1 (MiniMed, Inc.) provides an improved insertion set for transcutaneous placement of a sensor such as a glucose sensor at a selected site within the body of a patient. The insertion set comprises a mounting base defining an upwardly open channel for receiving and supporting a flexible thin film sensor, in combination with a cap assembled with said mounting base to capture and retain proximal end of the sensor within said channel.

### SUMMARY

The present disclosure provides an infusion device, the device comprising an ambulatory infusion device having a housing containing a fluid reservoir to contain a therapeutic fluid; a drive mechanism to drive the therapeutic fluid from the fluid reservoir ; a transcutaneous access tool fluidly coupled to the fluid reservoir, the transcutaneous access tool including at least a first cannula having at least a first lumen fluidly coupled with the reservoir and at least a deployable and retractable first needle/trocar located within the first lumen; a transcutaneous access tool insertion mechanism to deploy the transcutaneous access tool; the transcutaneous access tool configured to simultaneously introduce the first cannula subcutaneously and a monitoring test strip subcutaneously upon operation of the transcutaneous access tool insertion mechanism; and the transcutaneous access tool configured such that, when the therapeutic fluid from the fluid reservoir is delivered through the first lumen of the first cannula, the needle/trocar is contained within the first lumen of the first cannula.

In certain embodiments, an infusion device may comprise a fluid reservoir for containing a therapeutic fluid; and a transcutaneous access tool fluidly coupled to the fluid reservoir, which may deliver the therapeutic fluid subcutaneously and introduce a monitoring test strip subcutaneously.

In certain embodiments, a method to treat diabetes mellitus may be provided comprising providing an infusion device with integrated monitoring, with the device comprising a fluid reservoir for containing a therapeutic fluid; and a transcutaneous access tool fluidly coupled to the fluid reservoir, which may deliver the therapeutic fluid subcutaneously and introduce a monitoring test strip
subcutaneously; delivering the therapeutic fluid subcutaneously with the transcutaneous access tool to a patient, and introducing the monitoring test strip subcutaneously with the transcutaneous access tool to the patient.

In certain embodiments, the transcutaneous access tool includes a needle/trocar, and the transcutaneous access tool insertion mechanism is configured to insert and retract the needle/trocar in a single, uninterrupted motion. In such a manner, the pain of insertion and retraction of the needle/trocar experienced by the patient may be reduced.

In certain embodiments, the fluid delivery device may comprise a fluid reservoir; a transcutaneous access tool fluidly coupled to the fluid reservoir, the transcutaneous access tool including a needle/trocar; and a transcutaneous access tool insertion mechanism for deploying the transcutaneous access tool, wherein the insertion mechanism is configured to insert and retract the needle/trocar in a single, uninterrupted motion.

In certain embodiments, the fluid delivery device may comprise a fluid reservoir; a transcutaneous access tool fluidly coupled to the fluid reservoir, the transcutaneous access tool including at least a needle/trocar; and a transcutaneous access tool insertion mechanism for deploying the transcutaneous access tool, wherein the insertion mechanism is configured to insert the needle/trocar with an increasing insertion force as the needle/trocar moves in an insertion direction.

In certain embodiments, the transcutaneous access tool insertion mechanism for deploying a transcutaneous access tool including a cannula and a needle/trocar located inside of the cannula may comprise a first sliding member configured to move the needle/trocar in an insertion direction and a retraction direction; a second sliding member configured to move the cannula in the insertion direction; a torsion spring; and linkages coupled between the torsion spring and the first sliding member such that energy stored in the torsion spring causes the linkages to move the first sliding member in the insertion direction and the retraction direction.

In certain embodiments, the drive mechanism may comprise a clutch mechanism. As explained herein, by using a clutch mechanism, the number of fluid path prime pulses to prime the pump may be reduced and a full and proper priming of the fluid path before placement on the body may be better assured. The clutch mechanism may also be made suitable for other drug applications without significant redesign, and be more easily inspected than conventional drive mechanisms for infusion devices.

In certain embodiments, the fluid delivery device may comprise a fluid reservoir; a transcutaneous access tool fluidly coupled to the fluid reservoir; and a drive mechanism for driving fluid from the reservoir. The drive mechanism may comprise a plunger received in the reservoir; a leadscrew extending from the plunger; a nut threadably engaged with the leadscrew; a drive wheel; and a clutch mechanism coupled to the drive wheel, wherein the clutch mechanism is configured to allow the nut to pass through the clutch mechanism when disengaged and is configured to grip the nut when engaged such that the drive wheel rotates the nut to advance the leadscrew and the plunger into the reservoir.

In certain embodiments, the fluid delivery device may comprise a fluid reservoir; a transcutaneous access tool fluidly coupled to the fluid reservoir; and a drive mechanism for driving fluid from the reservoir The drive mechanism may comprise a plunger received in the reservoir; an elongated assembly comprising a first elongated member and a second elongated member; the first elongated member extending from the plunger; the second elongated member coupled to the first elongated member; a drive wheel; and a clutch mechanism coupled to the drive wheel, wherein the clutch mechanism is configured to allow the second elongated member to pass through when disengaged and is configured to grip the second elongated member when engaged such that the drive wheel rotates the second elongated member to advance the first elongated member and the plunger into the reservoir.

In certain embodiments, a method of operating a foregoing fluid delivery device may comprise providing the fluid delivery device; holding the clutch mechanism in a disengaged position; filling the fluid reservoir with fluid; passing the second elongated member through the clutch mechanism such that the plunger is retracted within the reservoir; releasing the clutch mechanism from the disengaged position; and engaging the clutch mechanism with the second elongated member.

### BRIEF DESCRIPTION OF THE DRAWINGS

These and other features and advantages will be better understood by reading the following detailed description, taken together with the drawings wherein:
FIG. 1 is a top perspective view of a fluid delivery device with a transcutaneous access tool insertion mechanism in a pre-deployment position, consistent with the present disclosure;
FIG. 2 is a bottom perspective view of a needle and cannula retracted into the fluid delivery device in the pre-deployment position shown in FIG. 1;
FIG. 3 is a top perspective view of the fluid delivery device shown in FIG. 1 with the insertion mechanism in an intermediate position;
FIG. 4 is a bottom perspective view of the needle and cannula extending from the fluid delivery device in the intermediate position shown in FIG. 3;
FIG. 5 is a top perspective view of the fluid delivery device shown in FIG. 1 with the insertion mechanism in a post-deployment position;
FIG. 6 is a bottom perspective view of the cannula extending from the fluid delivery device in the post-deployment position shown in FIG. 5;
FIG. 7 is a side perspective view of another embodiment of the insertion mechanism, consistent with the present disclosure, in a pre-deployment position;
FIG. 8 is a side perspective view of the insertion mechanism shown in FIG. 7 in an intermediate position;
FIG. 9 is a side perspective view of the insertion mechanism shown in FIG. 7 in a post-deployment position;
FIG. 10 is a top perspective view of the second sliding member of the insertion mechanism shown in FIG. 7 locked in the pre-deployment and post-deployment positions;
FIG. 11 is a top perspective view of a fluid driving mechanism of the fluid delivery device shown in FIG. 1 with a clutch mechanism in a disengaged position prior to filling;
FIG. 12 is a side cross-sectional view of the fluid driving mechanism shown in FIG. 11;
FIG. 13 is a top perspective view of the fluid driving mechanism shown in FIG. 11 with the clutch mechanism in a disengaged position after filling;
FIG. 14 is a top perspective view of the fluid driving mechanism shown in FIG. 11 with the clutch mechanism being released to the engaged position; and
FIGS. 15 and 16 are top perspective views of the fluid driving mechanism shown in FIG. 11 with the clutch mechanism in the engaged position.
FIGS. 17-23 are views of a bi-lumen cannula used in the fluid delivery device shown in FIGS. 1-6 to insert a monitor test strip transcutaneously;
FIGS. 24-29 are views of another embodiment of a fluid delivery device including a cannula with a D-shaped lumen for inserting a monitor test strip transcutaneously;
FIGS. 30-32 are views of the D-lumen cannula used in the fluid delivery device of FIGS. 24-29;
FIGS. 33 and 34 are views of a semi-circular trocar used with the D-lumen cannula in the fluid delivery device of FIGS. 18-23;
FIGS. 35-41 are views of another embodiment of a fluid delivery device including an oval trocar for inserting a monitor test strip transcutaneously;
FIG. 42 is a side view of the oval trocar for use in the fluid delivery device shown in FIGS. 35-41;
FIG. 43 is a top perspective view of a second sliding member for use in the fluid delivery device shown in FIGS. 35-41.

### DETAILED DESCRIPTION

A fluid delivery device, consistent with embodiments of the present disclosure, may be used to deliver a therapeutic fluid (e.g. a liquid medicine) to a patient via a transcutaneous access tool, such as a needle/trocar and/or a cannula. A transcutaneous access tool insertion mechanism may be used to deploy the transcutaneous access tool, for example, by inserting and retracting a needle/trocar in a single, uninterrupted motion. The insertion mechanism may also provide an increasing insertion force as the needle/trocar moves in the insertion direction. The fluid delivery device may also include a clutch mechanism to facilitate filling a reservoir and engagement of a drive mechanism for driving fluid out of the reservoir. In certain embodiments, the fluid delivery device may comprise an ambulatory insulin infusion device.

In other embodiments, a fluid delivery device may be used to deliver a therapeutic fluid to a patient with integrated monitoring, such as continuous glucose monitoring (CGM). In these embodiments, the fluid deliver device may include a transcutaneous access tool configured to introduce a monitoring test strip through the skin of the patient, for example, using one or more needles, cannulas and/or trocars.

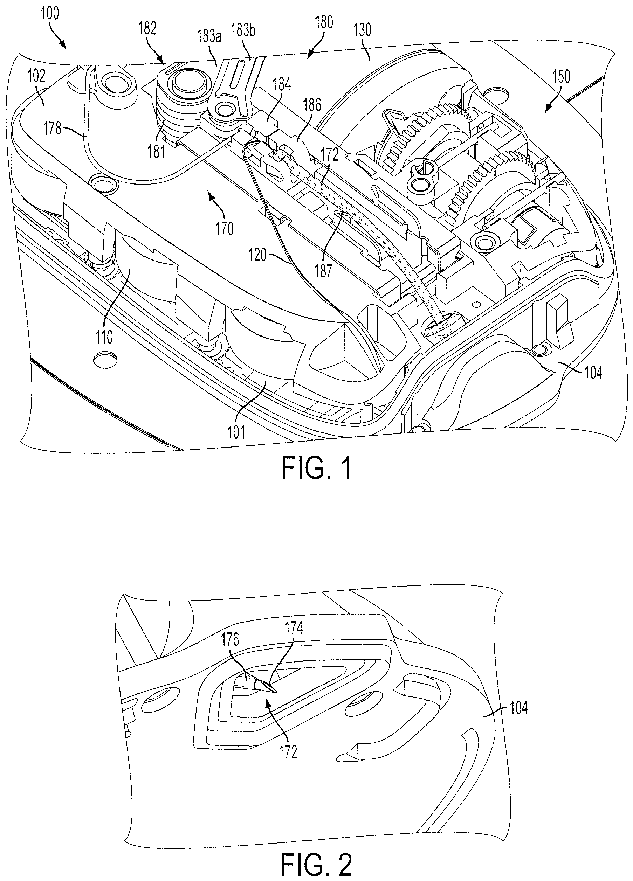

Referring to FIGS. 1-6, one embodiment of a fluid delivery device 100 is shown and described. In the exemplary embodiment, the fluid delivery device 100 is used to subcutaneously deliver a fluid, such as a liquid medicine (e.g. insulin), to a person or an animal. Those skilled in the art will recognize that the fluid delivery device 100 may be used to deliver other types of fluids. The fluid delivery device 100 may be used to deliver fluids in a controlled manner, for example, according to fluid delivery profiles accomplishing bolus requirements, continuous infusion and variable flow rate delivery.

According to one embodiment, the fluid delivery device 100 may include one or more batteries 110 for providing a power source, a fluid reservoir 130 for holding a fluid, a fluid drive mechanism 150 for driving the fluid out of the reservoir 130, a fluid passage mechanism 170 for receiving the fluid from the reservoir 130 and passing the fluid to a destination via a transcutaneous access tool 172, and a transcutaneous access tool insertion mechanism 180 for deploying the transcutaneous access tool 172. The fluid delivery device 100 may include a circuit board 101 with control circuitry for controlling the device and a chassis 102 that provides mechanical and/or electrical connections between components of the fluid deliver device 100. The fluid delivery device 100 may also include a housing 104 to enclose the circuit board 101, the chassis 102, and the components 110, 130, 150, 170, 180.

The fluid delivery device 100 may also include integrated monitoring such as continuous glucose monitoring (CGM). A monitor test strip 120 coupled to a monitor (not shown) in the device 100 may be introduced by the transcutaneous access tool 172 subcutaneously. One example of the monitor test strip is a CGM test strip (such as the type available from Nova Biomedical) which may be understood as a glucose sensor configured to test for a concentration level of glucose in the blood of a patient. The fluid delivery device 100 may be configured to receive data from the monitoring test strip concerning a glucose level of the patient, and determining an output of insulin from the reservoir based on the glucose level.

The transcutaneous access tool 172 includes an introducer trocar or needle 174 at least partially positioned within a lumen 175 of a cannula 176 (e.g., a soft flexible cannula), which is capable of passing the fluid into the patient. In particular, the introducer needle/trocar 174 may initially penetrate the skin such that both the introducer needle/trocar 174 and the cannula 176 are introduced (inserted) into the patient, and the introducer needle/trocar 174 may then be retracted within the cannula 176 such that the cannula 176 remains inserted. A fluid path, such as tubing 178, fluidly couples the reservoir 130 to the lumen 175 of cannula 176 of the transcutaneous access tool 172. The transcutaneous access tool 172 may also be used to introduce a monitoring test strip subcutaneously into the patient for monitoring purposes, as described in greater detail below.

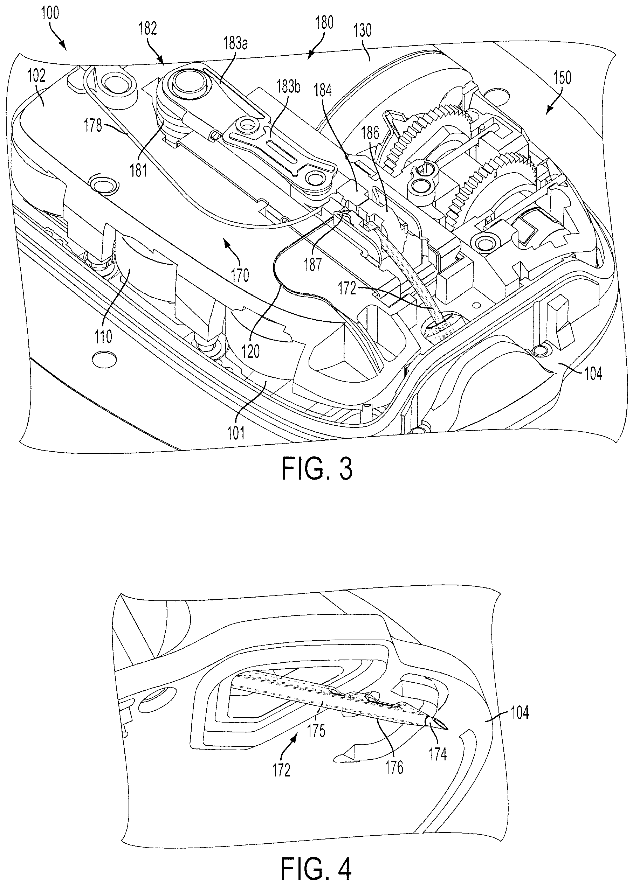

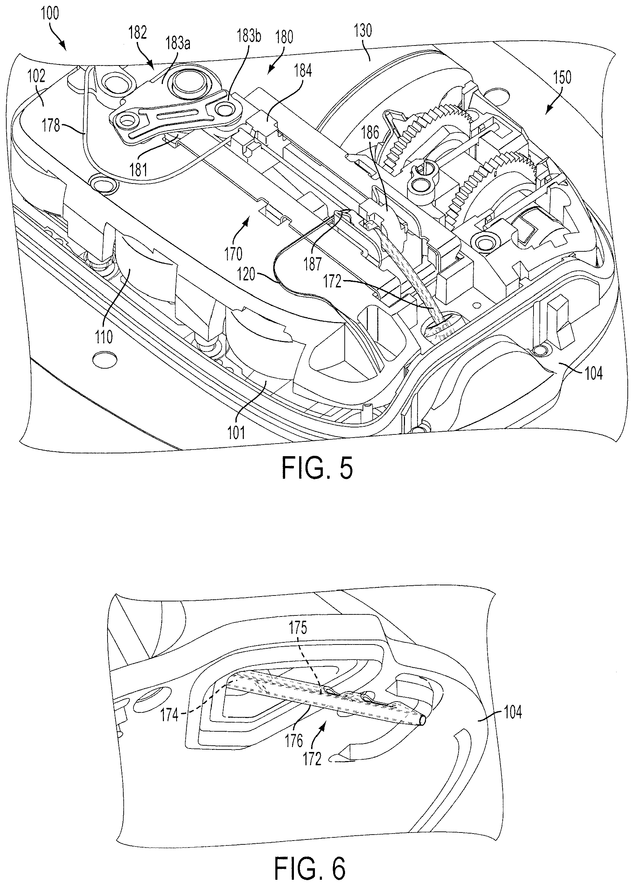

The transcutaneous access tool insertion mechanism 180 is coupled to the transcutaneous access tool 172 to deploy the transcutaneous access tool 172, for example, by inserting the needle/trocar 174 and cannula 176 through the skin of a patient and retracting the needle/trocar 174. In the illustrated embodiment, the insertion mechanism 180 includes a spring-biased linkage mechanism 182 and sliding members 184, 186 coupled to the needle/trocar 174 and cannula 176, respectively, for moving the needle/trocar 174 and cannula 176 in the insertion direction and for moving the needle/trocar 174 in the retraction direction. In a single, uninterrupted motion, the spring-biased linkage mechanism 182 moves from a pre-deployment position (FIG. 1) with both needle/trocar 174 and cannula 176 retracted (FIG. 2) to an intermediate position (FIG. 3) with both needle/trocar 174 and cannula 176 inserted (FIG. 4) to a post-deployment position (FIG. 5) with the needle/trocar 174 retracted and the cannula 176 inserted (FIG. 6).

One embodiment of the spring-biased linkage mechanism 182 includes a helical torsion spring 181 and first and second linkages 183a, 183b coupled between the torsion spring 181 and the first sliding member 184. Energy stored in the torsion spring 181 applies a force to the linkages 183a, 183b, which applies a force to the first sliding member 184 to move the first sliding member 184 in both the insertion direction and in the retraction direction. In the pre-deployment position (FIG. 1), the torsion spring 181 is loaded and the sliding members 184, 186 are locked and prevented from moving. When the sliding members 184, 186 are released, the energy stored in the torsion spring 181 causes the first linkage 183a to rotate (e.g., clockwise as shown), which applies a force to the first sliding member 184 through the second linkage 183b causing the first sliding member 184 with the needle/trocar 174 to move (with the second sliding member 186) in the insertion direction. In the intermediate position (FIG. 3), the linkages 183a, 183b are fully extended with the needle/trocar 174 and cannula 176 being inserted, the second sliding member 186 is locked, and the remaining energy stored in the torsion spring 181 causes the first linkage 183a to continue to rotate, which applies an opposite force to the first sliding member 184 through the second linkage 183b causing the first sliding member 184 with the needle/trocar 174 to move in the retraction direction to the post-deployment position (FIG. 5). In the illustrated embodiment, the second sliding member 186 is locked against retraction by one or more latches 187. Thus, in the foregoing manner, the continuous uninterrupted clockwise rotation of first linkage 183a via the energy of torsion spring 181 provides the transcutaneous access tool insertion mechanism 180 with the ability to insert and retract the needle/trocar 174 in a single, uninterrupted motion.

The spring-biased linkage mechanism 182 allows a single spring and motion to achieve both the insertion and retraction and has a relatively small size. The spring-biased linkage mechanism 182 also reduces the static stresses caused by locking and holding back the sliding members 184, 186 and provides a smoother and more comfortable needle/trocar insertion because of the way the linkages 183a, 183b vector the forces applied to the sliding members 184, 186. The static forces on the sliding members 184, 186 are relatively small in the pre-deployment position when the linkages 183a, 183b are fully retracted. When the deployment starts and the linkages 183a, 183b start to become extended, the insertion forces increase because the force vectors increase in the insertion direction as the linkages extend 183a, 183b until a maximum insertion force is reached at the fully extended, intermediate position. By gradually increasing the insertion forces, the needle/trocar insertion and retraction is smoother, quieter and less painful.

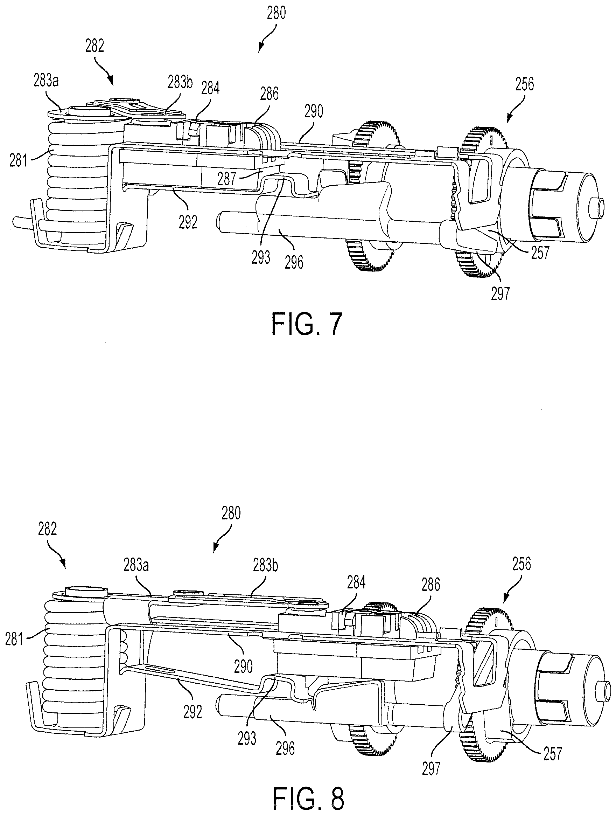

Another embodiment of an insertion mechanism 280 is shown in greater detail in FIGS. 7-10. The sliding members 284, 286 are slidably received in a frame 290 and moved by a spring-biased linkage mechanism 282 including torsion spring 281 and linkages 283a, 283b. In this embodiment, a cam finger 292 (e.g., extending from the frame 290) engages beneath one or both of the sliding members 284, 286 to lock the sliding members in the retracted or pre-deployment position (FIG. 7). In this pre-deployment position, the cam finger 292 is held against the sliding members 284, 286 by a release bar 296, which may be moved (rotated) to allow the cam finger 292 to move and release the sliding members 284, 286 (FIG. 8). The cam finger 292 may be biased in a downward direction and/or the second sliding member 286 may include a cam surface 287 to help facilitate movement along the cam finger 292 over locking mechanism 293 upon actuation.

The release bar 296 includes a lever 297 for pivoting the release bar 296 between an engaged position against the cam finger 292 (FIG. 7) and a disengaged position releasing the cam finger 292 (FIG. 8). The release bar 296 may be biased toward the disengaged position and held against the cam finger 292 in the engaged position until the lever 297 is released allowing the release bar 296 to move to the disengaged position. In the illustrated embodiment, the lever 297 engages a rotating surface 257 of a drive wheel 256 of the fluid drive mechanism 150 such that the lever 297 is held in the engaged position for part of the rotation and is released at a certain point during the rotation (e.g., when a flat portion of the rotating surface 257 allows the lever 297 to move).

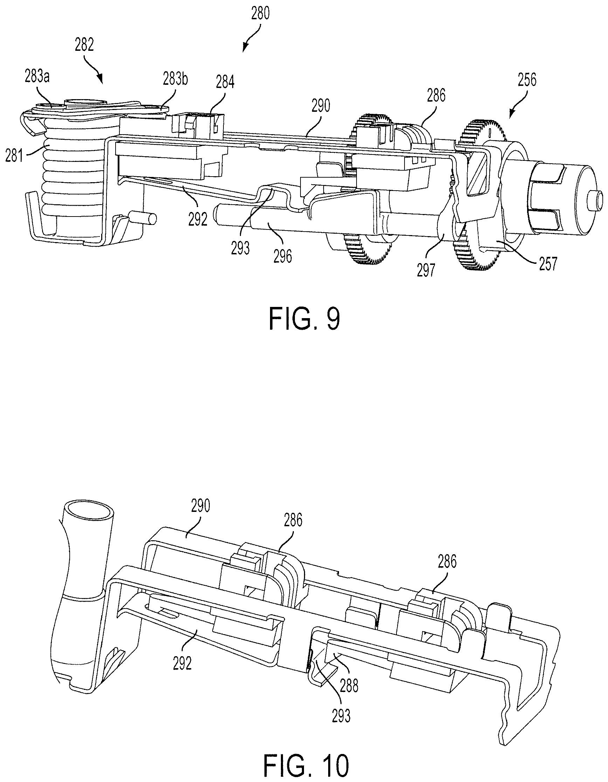

As shown in FIGS. 9 and 10, the cam finger 292 may also be used to lock the second sliding member 286 in the insertion position. A locking portion 288 of the second sliding member 286 engages a locking portion 293 of the cam finger 292 when the linkage mechanism 282 is fully extended in the intermediate position and prevents the second sliding member 286 from retracting such that the cannula remains inserted. As discussed above, the second sliding member 286 may also be locked by one or more latches (not shown) extending from a top of the frame 290.

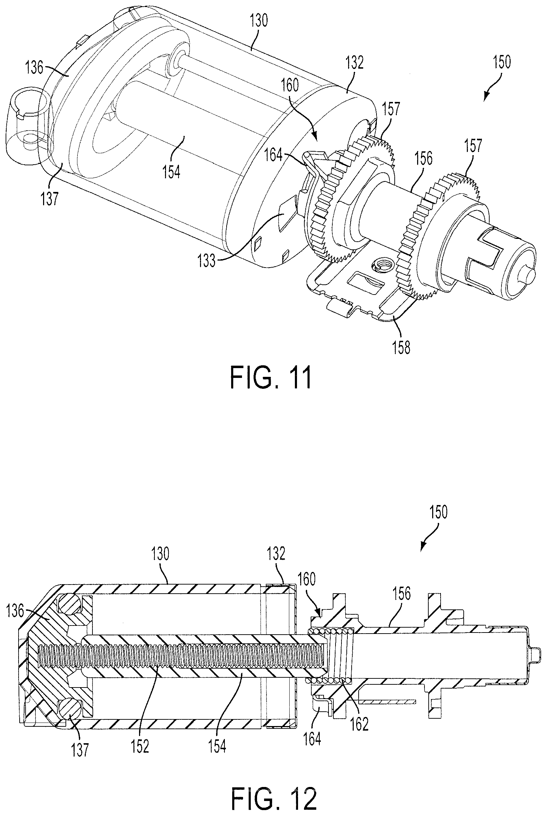

Referring to FIGS. 11-16, one embodiment of the fluid drive mechanism 150 uses a clutch mechanism 160 to facilitate filling of the reservoir 130 and engagement of the fluid drive mechanism 150 for driving fluid out of the reservoir 130. The fluid drive mechanism 150 includes a first threaded member in the form of an elongated shaft such as a threaded drive rod or leadscrew 152, with external threads extending from a plunger 136 received in the reservoir 130 and sealed with an o-ring 137 against the inside surface of the reservoir 130. The leadscrew 152 and plunger 136 may be an inseparable, insert-molded assembly. A second threaded member in the form of an elongated shaft such as a tube nut 154 with internal threads threadably engages the leadscrew 152 and may be driven by a drive wheel 156 via a clutch mechanism 160.

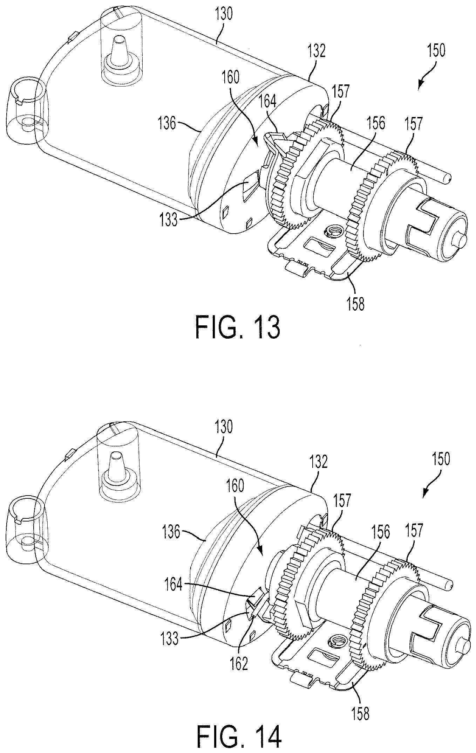

When the reservoir 130 is empty (FIGS. 11 and 12), the plunger 136 is positioned at one end of the reservoir 130 such that the plunger 136 is extended and the clutch mechanism 160 is disengaged. In certain embodiments, the reservoir 130 may be filled with fluid, particularly insulin, by opening an inlet port to the reservoir 130 and pumping in the insulin under sufficient hydraulic pressure to retract the plunger 136 within the reservoir 130. Thereafter, the inlet port may be closed. When the reservoir 130 is filled and the plunger 136 moves to the opposite (retracted) end of the reservoir 130 (FIG. 13), the clutch mechanism 160 remains disengaged to allow the tube nut 154 to pass into an elongated cylindrical bore (along the drive axis) of a hub of the drive wheel 156. The clutch mechanism 160 may then be engaged (FIGS. 14-16) such that rotation of the drive wheel 156 causes the clutch mechanism 160 to rotate the tube nut 154, which causes the leadscrew 152 to advance the plunger into the reservoir 130 to deliver the fluid from the reservoir 130. In alternative embodiments, the reservoir 130 may be filled when the plunger 136 is already retracted.

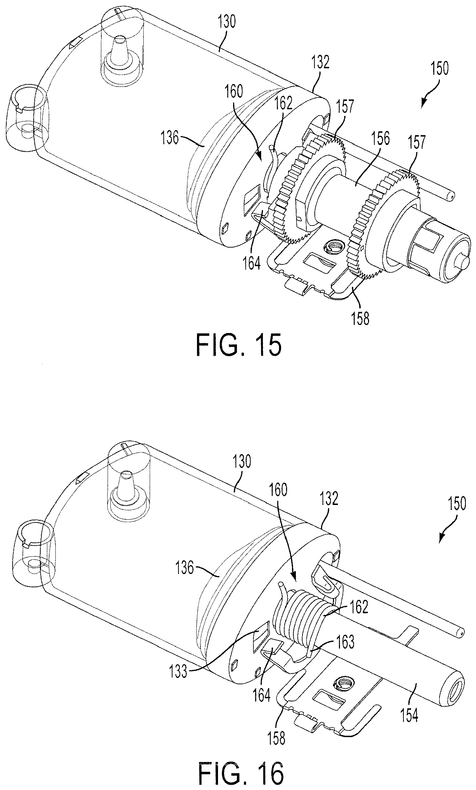

In the illustrated embodiment, the clutch mechanism 160 includes a clutch spring 162 (e.g., a helical torsion spring) located in a counterbore at one end of the drive wheel 156, adjacent the reservoir 130. The inside diameter of the clutch spring 162 is larger than the outside diameter of the tube nut 154 when the clutch spring 162 is loaded, thereby disengaging the clutch spring 162 from the tube nut 154 and allowing the tube nut 154 to pass through the center aperture of the spring 162 and into the elongated bore of the drive wheel 156. Alternatively, the inside diameter of the clutch spring 162 is smaller than the outside diameter of the tube nut 154 when the clutch spring 162 is unloaded, thereby engaging or gripping the tube nut 154 and allowing the drive wheel 156 to rotate the tube nut 154. In the illustrated embodiment, prior to filing the reservoir 130, the clutch spring 162 is held in the loaded, disengaged position by a spring latch 164 engaged with the drive wheel 156 (FIGS. 11-13). After the reservoir 130 has been filled, the clutch spring 162 may thus be engaged by rotating the drive wheel 156 until the spring latch 164 releases the clutch spring 162 (FIG. 14) allowing the clutch spring 162 to unload and grip the tube nut 154 (FIGS. 15 and 16), at which time fluid may be dispensed from the reservoir 130 with continued rotation of the drive wheel 156.

As shown, the spring latch 164 may be biased by the clutch spring 162 such that as the drive wheel 156 rotates the spring latch 164 moves rotationally against a surface of a reservoir cap 132 until clutch spring 162 deflects the spring latch 164 into a window 133 in the reservoir cap 132. When the spring latch 164 moves into the window 133, the end of the clutch spring 162 held by the spring latch 164 is released, thus engaging the clutch mechanism 160. When the clutch spring 162 is engaged, the drive wheel 156 contacts an end 163 of the clutch spring 162 to create a thrust on the clutch spring 162 that causes the clutch spring 162 to rotate the tube nut 154. The fluid drive mechanism 150 may also use other clutch mechanisms capable of allowing the tube nut 154 or other type of nut or threaded member to pass through the clutch mechanism and then being activated to engage the nut or threaded member.

In the illustrated embodiment, the drive wheel 156 includes ratchets 157 that are engaged by an actuator 158 to incrementally drive the wheel 156 and advance the plunger 136 into the reservoir 130. Examples of this actuation mechanism are described in greater detail in U.S. Patent Application Publication No. 2005/0238507, which is fully incorporated herein by reference.

By using a clutch mechanism, the engagement between the leadscrew and the nut occurs at assembly, and thus no rotation is needed for the nut to engage the leadscrew by operation of the device. This reduces the number of fluid path prime pulses to prime the pump and assures a full and proper priming of the fluid path before placement on the body. The clutch mechanism also enables the changing of thread pitch for other drug applications without a need to redesign the tilt nut used in fluid driving mechanisms in other existing pumps. The components of the clutch mechanism are also more easily inspected than the tilt nut assembly.

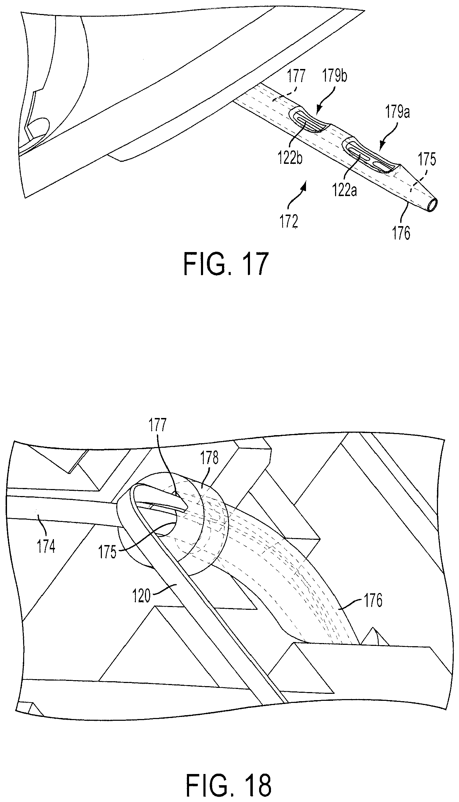

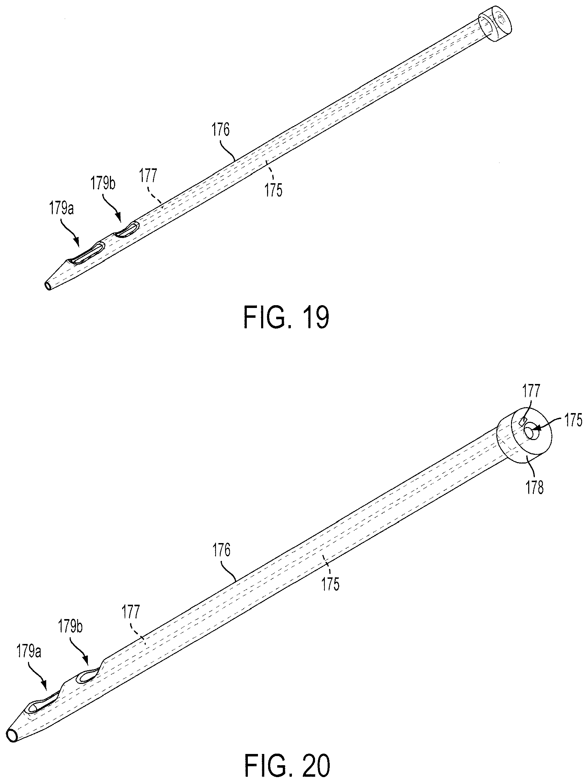

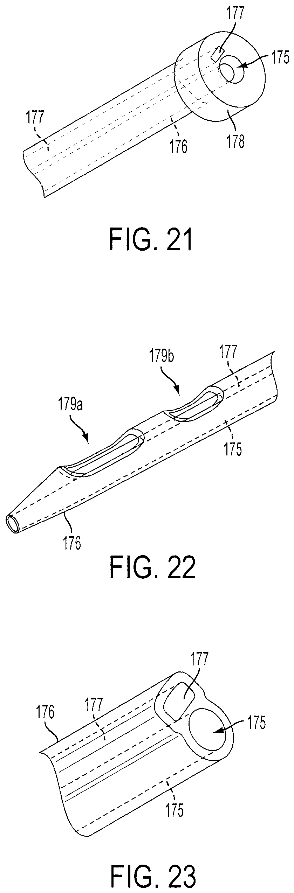

According to one embodiment, as shown in FIGS. 17-23, the cannula 176 providing the transcutaneous access for delivery the fluid may also be used to introduce the monitor test strip 120. In this embodiment, the cannula 176 includes a first lumen 175 for receiving the needle/trocar 174 and a second lumen 177 for receiving the test strip 120. As shown, the first lumen 175 has a circular (cylindrical) profile and the second lumen 177 has a rectangular profile. The cannula 176 may also include one or more windows 179a, 179b providing access to one or more sensors 122a, 122b on the test strip 120. As shown, the plurality of windows 179a, 179b of the cannula 176 may be arranged on a same side of the sidewall of cannula 176, with the first window 179a arranged at a distance from the distal end tip of the cannula 176 which is less than the distance of the second window 179b from the distal end tip of the cannula 176.

To insert the test strip 120 into second lumen 177, the test strip 120 passes into second lumen 177 at the head 178 of the cannula 176 and extends to the window(s) 179a, 179b. Thus, at least one window 179a, 179b exposes a sensor 122a, 122b of the monitoring test strip 120. In the example embodiment, two windows 179a, 179b are provided with the window 179a closest to the tip of the cannula 176 providing access to the main sensor area and the window 179b farthest from the tip providing a reference. Although a specific shape and configuration of a bi-lumen cannula is shown, other configurations of a cannula with first and second lumens may also be used to both deliver a therapeutic fluid and introduce a test strip subcutaneously.

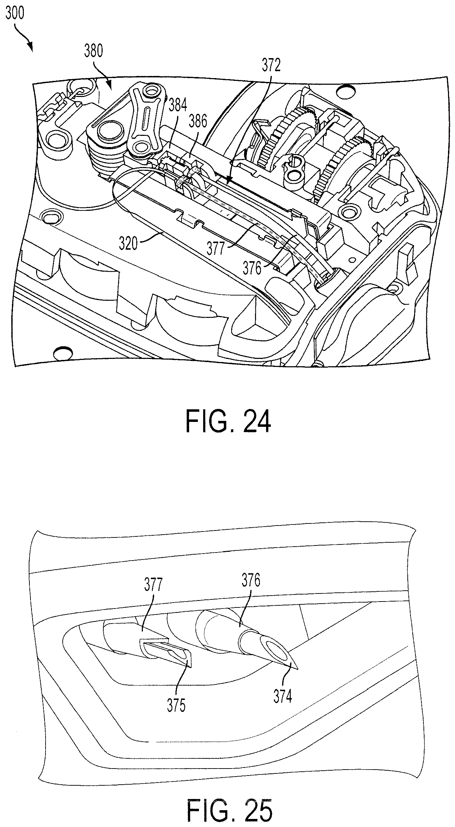

According to another embodiment, as shown in FIGS. 24-34, a fluid delivery device 300 may include a transcutaneous access tool 372 with a first cannula 376 for delivering fluid and a second cannula 377 for introducing a test strip 320. The first cannula 376 receives a first needle/trocar 374 (shown as a circular needle) to facilitate insertion of the first cannula 376 and the second cannula 377 receives a second needle/trocar 375 (shown as a semi-circular trocar) to facilitate insertion of the second cannula 377. The fluid deliver device 300 includes an insertion mechanism 380, similar to the first described embodiment above, but with sliding members 384, 386 coupled to both the needle 374 and the trocar 375 and both cannulas 376, 377. The insertion mechanism 380 inserts the second cannula 377 and the trocar 375 and then retracts the trocar 375 in the same manner as described above. The test strip 320 remains inserted after the trocar 375 is retracted. Thus, both the first needle/trocar 374 and the second needle/trocar 375 may be introduced into the patient simultaneously, particularly to reduce the pain of sequential insertions.

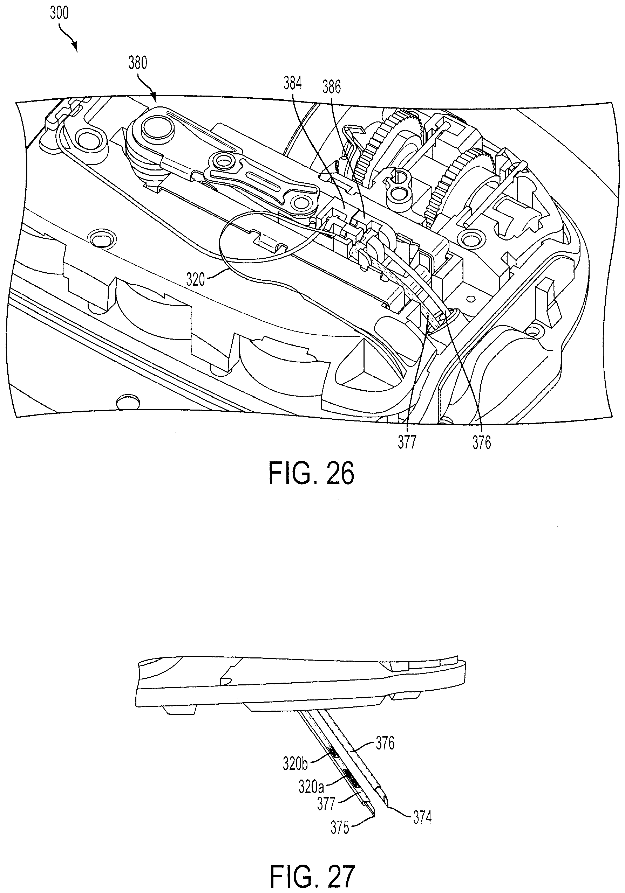

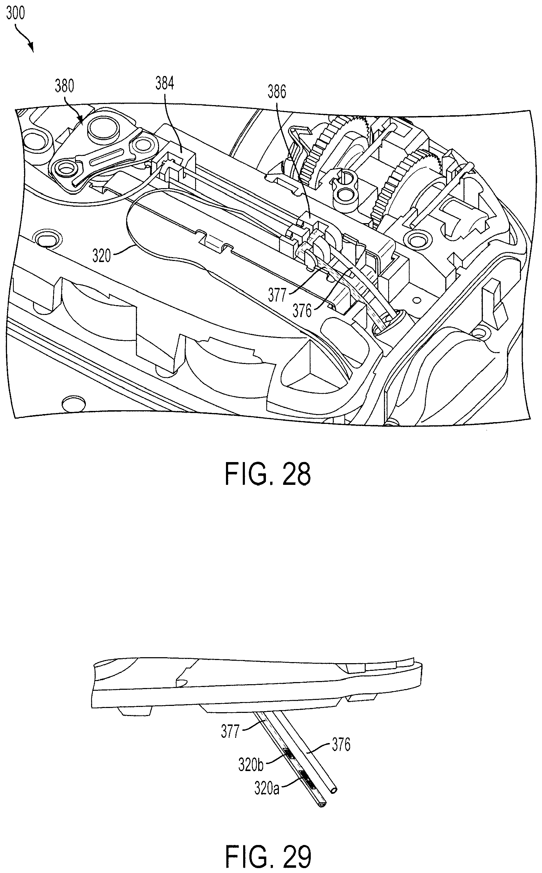

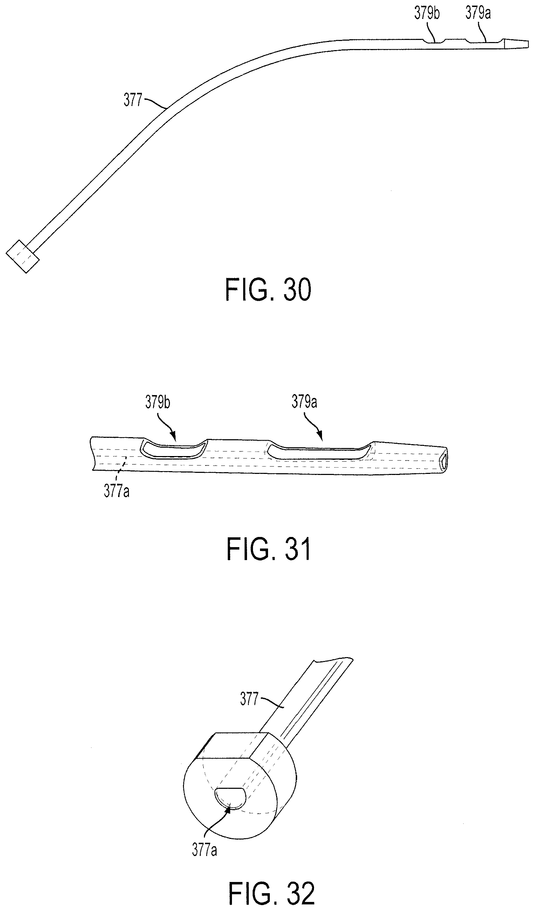

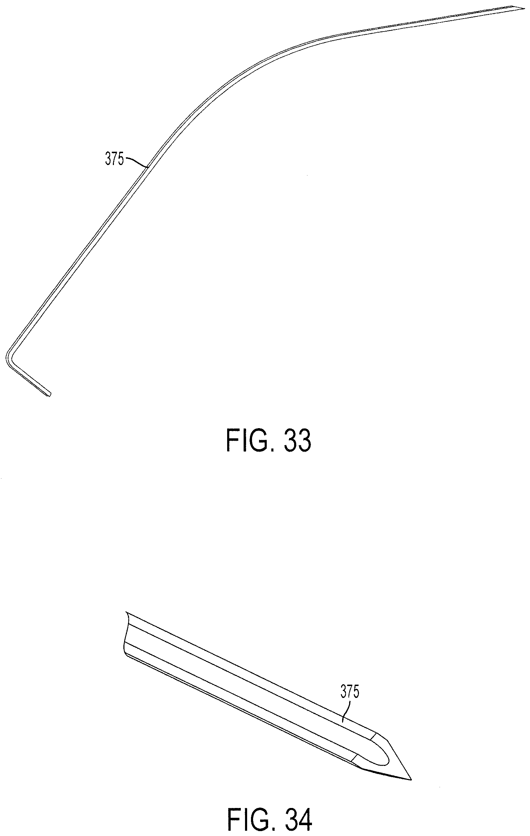

Similar to the above described embodiment, first cannula 376 includes a circular (cylindrical) lumen 376a. As shown in greater detail in FIGS. 30-32, the second cannula 377 includes a semi-circular (D-shaped) lumen 377a to allow the monitor strip to sit relatively flat within the cannula 377. The second cannula 377 also includes one or more windows 379a, 379b providing access to one or more sensors 320a, 320b on the test strip 320 (see FIGS. 27 and 29). As shown, similar to the prior embodiment, the plurality of windows 379a, 379b, of the cannula 377 may be arranged on a same side of the sidewall of the cannula 377, with the first window 379a arranged at a distance from the distal end tip of the cannula 377 which is less than the distance of the second window 379b from the distal end tip of the cannula 377. Thus, at least one window 379a, 379b exposes a sensor 320a, 320b of the monitoring test strip 320. In the example embodiment, two windows 379a, 379b are provided with the window 379a closest to the tip of the cannula 377 providing access to the main sensor area and the window 379b farthest from the tip providing a reference. As shown in greater detail in FIGS. 33 and 34, the trocar 375 has a shape corresponding to the D-shaped lumen 377a to allow the trocar 375 to be retracted leaving the test strip 320 inserted (see FIG. 29). As shown, the trocar includes a planar side surface 373 which corresponds to a planar test strip 320 such that, when assembled, the planar test strip 320 may be located adjacent the planar side surface 373 of the trocar 375 in the second cannula 377.

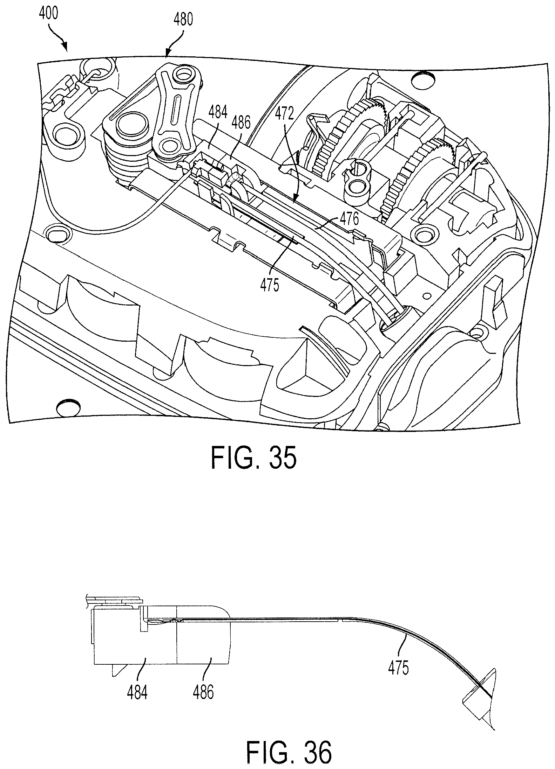

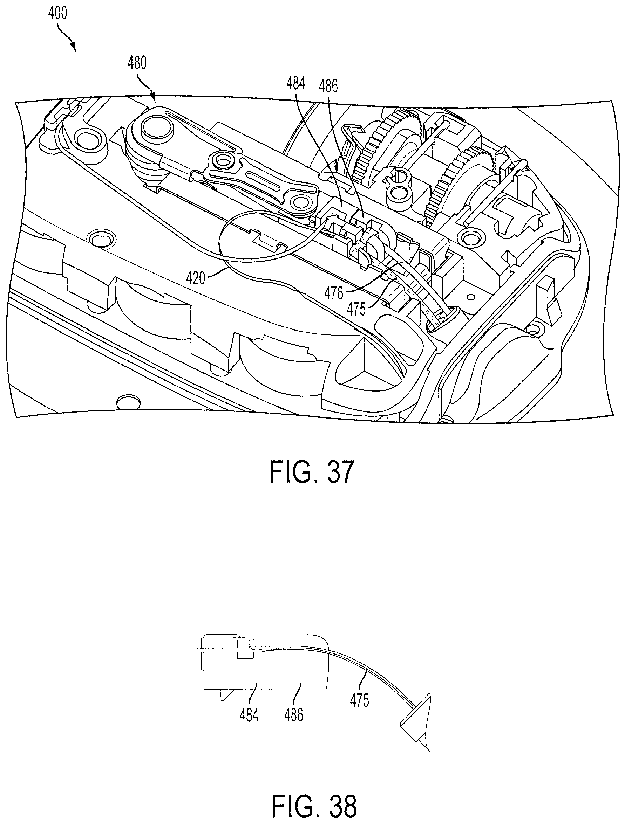

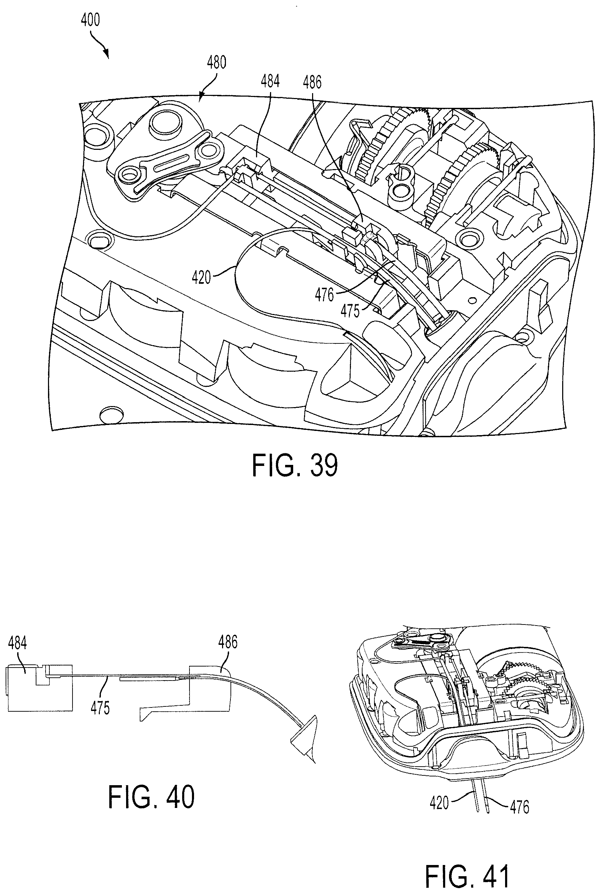

According to another embodiment, as shown in FIGS. 35-43, a fluid delivery device 400 may include a transcutaneous access tool 472 with a cannula 476 for delivering fluid and a needle or trocar 475 (shown as a semi-circular trocar) for introducing a test strip 420. The cannula 476 receives a needle/trocar 474 (shown as circular needle) to facilitate insertion of the cannula 476 and the trocar 475 is inserted with the test strip 420. The fluid deliver device 400 includes an insertion mechanism 480, similar to the first described embodiment above, but with sliding members 484, 486 coupled to both the needle 474 and the trocar 475. The insertion mechanism 480 inserts the trocar 475 (FIGS. 37 and 38) and then retracts the trocar 475 (FIGS. 39 and 40) in the same manner as the needle/trocar described above. The test strip 420 remains inserted after the trocar 475 is retracted (FIG. 41). In contrast to the prior embodiment, the needle/trocar 475 introduces the monitoring test strip 420 subcutaneously solely (i.e. without the monitoring test strip 420 being introduced with a cannula).

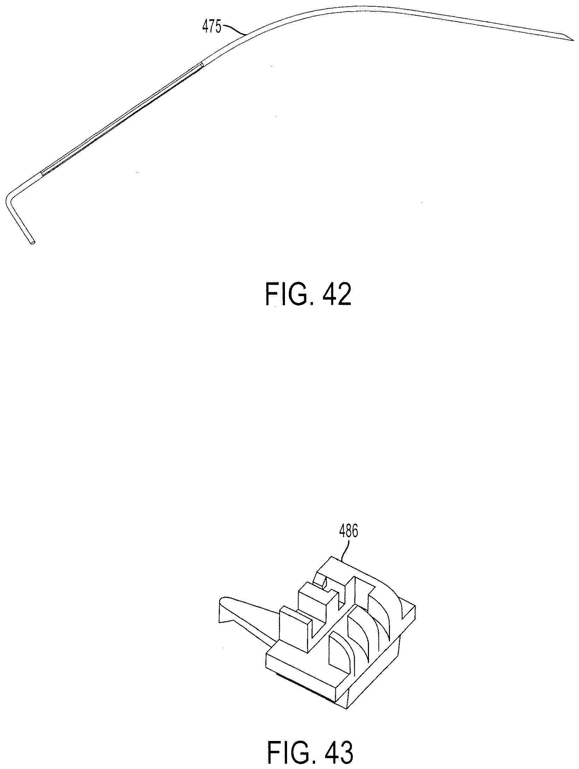

The trocar 475 is shown in greater detail in FIG. 42. The second sliding member 486 is shown in greater detail in FIG. 43. In this embodiment, the second sliding member 486 is designed to capture the cannula 476 and to receive and allow the trocar 475 to pass through.

Accordingly, various embodiments of the fluid delivery device may use the transcutaneous access tool both to deliver fluid and to introduce a test strip subcutaneously to provide integrated monitoring.

While the principles of the invention have been described herein, it is to be understood by those skilled in the art that this description is made only by way of example and not as a limitation as to the scope of the invention. Other embodiments are contemplated within the scope of the present invention in addition to the exemplary embodiments shown and described herein. Modifications and substitutions by one of ordinary skill in the art are considered to be within the scope of the present invention, which is not to be limited except by the following claims.

## Claims

1. An infusion device (100, 300, 400), the device comprising:
an ambulatory infusion device having a housing (104) containing
a fluid reservoir (130) to contain a therapeutic fluid;
a drive mechanism (150) to drive the therapeutic fluid from the fluid reservoir (130);
a transcutaneous access tool (172) fluidly coupled to the fluid reservoir (130), the transcutaneous access tool (172) including at least a first cannula (176, 376, 476) having at least a first lumen (175) fluidly coupled with the reservoir (130) and at least a deployable and retractable first needle/trocar (174, 374, 475) located within the first lumen (175);
a transcutaneous access tool (172) insertion mechanism (180, 280) to deploy the transcutaneous access tool (172);
**characterized in that** the transcutaneous access tool (172) is configured to simultaneously introduce the first cannula (176, 376, 476) subcutaneously and a monitoring test strip (120, 320, 420) subcutaneously upon operation of the transcutaneous access tool (172) insertion mechanism (180, 280); and
the transcutaneous access tool (172) configured such that, when the therapeutic fluid from the fluid reservoir (130) is delivered through the first lumen (175) of the first cannula (176, 376, 476), the needle/trocar (174, 374, 475) is contained within the first lumen (175) of the first cannula (176, 376, 476).

2. The infusion device of claim 1 wherein:
the monitoring test strip is located in a second lumen of the first cannula.

3. The infusion device of claim 1 wherein:
the transcutaneous access tool includes a second cannula and a second needle/trocar;
the second needle/trocar passes through a lumen of the second cannula;and the monitoring test strip is located in the lumen of the second cannula.

4. The infusion device of claim 1 wherein:
the transcutaneous access tool includes a second needle/trocar;
the second needle/trocar is configured to introduce the monitoring test strip subcutaneously.

5. The infusion device of claim 1 wherein:
the transcutaneous access tool insertion mechanism is configured to at least one of:
insert and retract the first needle/trocar in a single, uninterrupted motion; and
insert the first needle/trocar with an increasing insertion force as the first needle/trocar moves in an insertion direction.

6. The infusion device of claim 5 wherein:
the first needle/trocar is located within the first cannula such that the first cannula remains inserted when the first needle/trocar is retracted.

7. The infusion device of claim 6 wherein:
the transcutaneous access tool insertion mechanism comprises
a first sliding member configured to move the first needle/trocar in an insertion direction and in a retraction direction;
a second sliding member configured to move the first cannula in the insertion direction;
a torsion spring; and
linkages coupled between the torsion spring and the first sliding member such that energy stored in the torsion spring causes the linkages to move the first sliding member in the insertion direction and the retraction direction.

8. The infusion device of claim 7 wherein:
the transcutaneous access tool insertion mechanism further comprises
a frame slidably receiving the first and second sliding members and configured to lock the first and second sliding members in a pre-deployment position and to lock the second sliding member in a post-deployment position.

9. The infusion device of claim 8 wherein:
the frame includes a cam finger configured to lock the first and second sliding members in the pre-deployment position.

10. The infusion device of claim 9 wherein:
the cam finger is configured to lock the second sliding member in the post-deployment position.

11. The infusion device of claim 9 wherein:
the transcutaneous access tool insertion mechanism further comprises
a release bar configured to hold the cam finger when the cam finger locks the first and second sliding members in the pre-deployment position and configured to release the cam finger to allow the first and second sliding members to move in the insertion direction.

12. The infusion device of claim 11 wherein:
the drive mechanism engages the release bar and triggers movement of the release bar to release the cam finger.

13. The infusion device of claim 1 wherein:
the drive mechanism comprises
a plunger received in the reservoir;
an elongated assembly comprising a first elongated member and a second elongated member;
the first elongated member extending from the plunger;
the second elongated member coupled to the first elongated member;
a drive wheel; and
a clutch mechanism coupled to the drive wheel, wherein the clutch mechanism is configured to allow the second elongated member to pass through when disengaged and is configured to grip the second elongated member when engaged such that the drive wheel rotates the second elongated member to advance the first elongated member and the plunger into the reservoir.

14. The infusion device of claim 13 wherein:
the first elongated member comprises a first threaded member;
the second elongated member comprises a second threaded member;
the second threaded member is in threaded engagement with the first threaded member; and
the clutch mechanism is configured to allow the second threaded member to pass through when disengaged and is configured to grip the second threaded member when engaged such that the drive wheel rotates the second threaded member to advance the first threaded member and the plunger into the reservoir.

15. The infusion device of claim 14 wherein:
the first elongated threaded member comprises a first threaded shaft having external threads.

16. The infusion device of claim 14 wherein:
the second elongated threaded member comprises a second threaded shaft having internal threads.

17. The infusion device of claim 13 wherein:
the clutch mechanism includes a clutch spring that grips the second elongated member when released.

18. The infusion device of claim 17 wherein:
the clutch mechanism further includes a spring latch configured to hold the clutch spring in a disengaged position and configured to release the clutch spring such that the clutch spring moves to an engaged position.

19. The infusion device of claim 18 wherein:
the spring latch is configured to release the clutch spring in response to movement of the drive wheel.

## Patentansprüche

1. Infusionsvorrichtung (100, 300, 400), wobei die Vorrichtung umfasst:
eine ambulante Infusionsvorrichtung mit einem Gehäuse (104), enthaltend
ein Fluidreservoir (130), um ein therapeutisches Fluid zu enthalten;
einen Antriebsmechanismus (150), um das therapeutische Fluid aus dem Fluidspeicher (130) anzutreiben;
ein transkutanes Zugangswerkzeug (172), das fluidisch mit dem Fluidreservoir (130) gekoppelt ist, wobei das transkutane Zugangswerkzeug (172) mindestens eine erste Kanüle (176, 376, 476) mit mindestens einem ersten Lumen (175), das fluidisch mit dem Reservoir (130) gekoppelt ist, und mindestens eine(n) einsetzbare(n) und rückziehbare(n) erste(n) Nadel/Trokar (174, 374, 475), die(der) sich innerhalb des ersten Lumens (175) befindet, enthält;
einen transkutanen Zugangswerkzeug (172) -Einführungsmechanismus (180, 280), um das transkutane Zugangswerkzeug (172) einzusetzen;
**dadurch gekennzeichnet, dass** das transkutane Zugangswerkzeug (172) konfiguriert ist, um gleichzeitig die erste Kanüle (176, 376, 476) subkutan und einen Überwachungsteststreifen (120, 320, 420) subkutan nach Betätigen des Einführungsmechanismus (180, 280) für das transkutane Zugangswerkzeug (172) einzuführen; und
das transkutane Zugangswerkzeug (172) so konfiguriert ist, dass, wenn das therapeutische Fluid aus dem Fluidreservoir (130) durch das erste Lumen (175) der ersten Kanüle (176, 376, 476) abgegeben wird, die(der) Nadel/Trokar (174, 374, 475) innerhalb des ersten Lumens (175) der ersten Kanüle (176, 376, 476) enthalten ist.

2. Infusionsvorrichtung nach Anspruch 1, wobei:
sich der Überwachungsteststreifen in einem zweiten Lumen der ersten Kanüle befindet.

3. Infusionsvorrichtung nach Anspruch 1, wobei:
das transkutane Zugangswerkzeug eine zweite Kanüle und eine(n) zweite(n) Nadel/Trokar enthält;
die(der) zweite Nadel/Trokar durch ein Lumen der zweiten Kanüle durchläuft; und
sich der Überwachungsteststreifen im Lumen der zweiten Kanüle befindet.

4. Infusionsvorrichtung nach Anspruch 1, wobei:
das transkutane Zugangswerkzeug eine(n) zweite(n) Nadel/Trokar enthält;
die(der) zweite Nadel/Trokar konfiguriert ist, um den Überwachungsteststreifen subkutan einzuführen.

5. Infusionsvorrichtung nach Anspruch 1, wobei:
der transkutane Zugangswerkzeug-Einführungsmechanismus für mindestens eines der Folgenden konfiguriert ist:
Einführen und Rückziehen der(des) ersten Nadel/Trokars in einer einzigen, ununterbrochenen Bewegung; und
Einführen der(des) ersten Nadel/Trokars mit einer zunehmenden Einführungskraft, wenn sich die(der) erste Nadel/Trokar in einer Einführungsrichtung bewegt.

6. Infusionsvorrichtung nach Anspruch 5, wobei:
die(der) erste Nadel/Trokar innerhalb der ersten Kanüle angeordnet ist, so dass die erste Kanüle eingeführt bleibt, wenn die(der) erste Nadel/Trokar zurückgezogen wird.

7. Infusionsvorrichtung nach Anspruch 6, wobei:
der transkutane Zugangswerkzeug-Einführungsmechanismus ein erstes Gleitelement umfasst, das konfiguriert ist, um die(den) erste(n) Nadel/Trokar in eine Einführungsrichtung und in eine Rückzugsrichtung zu bewegen;
ein zweites Gleitelement, das konfiguriert ist, um die erste Kanüle in die Einführungsrichtung zu bewegen;
eine Torsionsfeder; und
Verbindungen, die zwischen der Torsionsfeder und dem ersten Gleitelement gekoppelt sind, so dass die in der Torsionsfeder gespeicherte Energie bewirkt, dass die Verbindungen das erste Gleitelement in Einführungsrichtung und Rückzugsrichtung bewegen.

8. Infusionsvorrichtung nach Anspruch 7, wobei:
der transkutane Zugangswerkzeug-Einführungsmechanismus weiter einen Rahmen umfasst, der das erste und zweite Gleitelement gleitend aufnimmt und konfiguriert ist, um das erste und zweite Gleitelement in einer Voreinsatzposition zu verriegeln und das zweite Gleitelement in einer Nacheinsatzposition zu verriegeln.

9. Infusionsvorrichtung nach Anspruch 8, wobei:
der Rahmen einen Nockenfinger enthält, der konfiguriert ist, um das erste und zweite Gleitelement in der Voreinsatzposition zu verriegeln.

10. Infusionsvorrichtung nach Anspruch 9, wobei:
der Nockenfinger konfiguriert ist, um das zweite Gleitelement in der Nacheinsatzposition zu verriegeln.

11. Infusionsvorrichtung nach Anspruch 9, wobei:
der transkutane Zugangswerkzeug-Einführmechanismus weiter eine Freigabestange umfasst, die konfiguriert ist, um den Nockenfinger zu halten, wenn der Nockenfinger das erste und zweite Gleitelement in der Voreinsatzposition verriegelt, und konfiguriert ist, um den Nockenfinger freizugeben, damit sich das erste und zweite Gleitelement in der Einführungsrichtung bewegen können.

12. Infusionsvorrichtung nach Anspruch 11, wobei:
der Antriebsmechanismus in die Freigebestange eingreift und eine Bewegung der Freigebestange auslöst, um den Nockenfinger freizugeben.

13. Infusionsvorrichtung nach Anspruch 1, wobei:
der Antriebsmechanismus einen Kolben umfasst, der in dem Reservoir aufgenommen ist;
eine längliche Anordnung, umfassend ein erstes längliches Element und ein zweites längliches Element;
wobei sich das erste längliche Element vom Kolben erstreckt;
das zweite längliche Element mit dem ersten länglichen Element gekoppelt ist;
ein Antriebsrad; und
einen Kupplungsmechanismus, der mit dem Antriebsrad gekoppelt ist, wobei der Kupplungsmechanismus konfiguriert ist, um das zweite längliche Element durchgehen zu lassen, wenn es ausgekoppelt ist, und konfiguriert ist, um das zweite längliche Element zu greifen, wenn es in Eingriff ist, so dass das Antriebsrad das zweite längliche Element dreht, um das erste längliche Element und den Kolben in dem Reservoir vorzubewegen.

14. Infusionsvorrichtung nach Anspruch 13, wobei:
das erste längliche Element ein erstes Gewindeelement umfasst;
das zweite längliche Element ein zweites Gewindeelement umfasst;
das zweite Gewindeelement in Gewindeeingriff mit dem ersten Gewindeelement steht;
und
der Kupplungsmechanismus konfiguriert ist, um das zweite Gewindeelement im ausgekoppelten Zustand durchlaufen zu lassen, und konfiguriert ist, um das zweite Gewindeelement im eingekoppelten Zustand so zu greifen, dass das Antriebsrad das zweite Gewindeelement dreht, um das erste Gewindeelement und den Kolben in dem Reservoir vorzubewegen.

15. Infusionsvorrichtung nach Anspruch 14, wobei:
das erste längliche Gewindeelement einen ersten Gewindeschaft mit Außengewinden umfasst.

16. Infusionsvorrichtung nach Anspruch 14, wobei:
das zweite längliche Gewindeelement einen zweiten Gewindeschaft mit Innengewinden umfasst.

17. Infusionsvorrichtung nach Anspruch 13, wobei:
der Kupplungsmechanismus eine Kupplungsfeder enthält, die, wenn freigegeben, das zweite längliche Element greift.

18. Infusionsvorrichtung nach Anspruch 17, wobei:
der Kupplungsmechanismus weiter eine Federverriegelung enthält, die konfiguriert ist, um die Kupplungsfeder in einer ausgekoppelten Position zu halten, und die konfiguriert ist, um die Kupplungsfeder so freizugeben, dass sich die Kupplungsfeder in eine Eingriffsposition bewegt.

19. Infusionsvorrichtung nach Anspruch 18, wobei:
die Federverriegelung konfiguriert ist, um die Kupplungsfeder als Reaktion auf die Bewegung des Antriebsrades freizugeben.

## Revendications

1. Dispositif de perfusion (100, 300, 400), le dispositif comprenant :
un dispositif de perfusion ambulatoire ayant un logement (104) contenant
un réservoir de fluide (130) pour contenir un fluide thérapeutique ;
un mécanisme d'entraînement (150) pour entraîner le fluide thérapeutique depuis le réservoir de fluide (130) ;
un outil d'accès transcutané (172) couplé fluidiquement au réservoir de fluide (130), l'outil d'accès transcutané (172) incluant au moins une première canule (176, 376, 476) ayant au moins une première lumière (175) couplée fluidiquement avec le réservoir (130) et au moins une première aiguille/un premier trocart déployable et rétractable (174, 374, 475) situé(e) à l'intérieur de la première lumière (175) ;
un mécanisme d'insertion (180, 280) d'outil d'accès transcutané (172) pour déployer l'outil d'accès transcutané (172) ;
**caractérisé en ce que** l'outil d'accès transcutané (172) est configuré pour introduire simultanément la première canule (176, 376, 476) par voie sous-cutanée et une bandelette réactive de contrôle (120, 320, 420) par voie sous-cutanée lors de l'actionnement du mécanisme d'insertion (180, 280) d'outil d'accès transcutané (172) ; et
l'outil d'accès transcutané (172) étant configuré de sorte que, lorsque le fluide thérapeutique provenant du réservoir de fluide (130) est administré à travers la première lumière (175) de la première canule (176, 376, 476), l'aiguille/trocart (174, 374, 475) soit contenu(e) à l'intérieur de la première lumière (175) de la première canule (176, 376, 476).

2. Dispositif de perfusion selon la revendication 1, dans lequel :
la bandelette réactive de contrôle est située dans une seconde lumière de la première canule.

3. Dispositif de perfusion selon la revendication 1, dans lequel :
l'outil d'accès transcutané inclut une seconde canule et une seconde aiguille/un second trocart ;
la seconde aiguille/le second trocart passe à travers une lumière de la seconde canule ; et
la bandelette réactive de contrôle est située dans la lumière de la seconde canule.

4. Dispositif de perfusion selon la revendication 1, dans lequel :
l'outil d'accès transcutané inclut une seconde aiguille/un second trocart ;
la seconde aiguille/le second trocart est configuré(e) pour introduire la bandelette réactive de contrôle par voie sous-cutanée.

5. Dispositif de perfusion selon la revendication 1, dans lequel :
le mécanisme d'insertion d'outil d'accès transcutané est configuré pour au moins l'un de :
insérer et rétracter la première aiguille/le premier trocart en un mouvement unique ininterrompu ; et
insérer la première aiguille/le premier trocart avec une force d'insertion accrue à mesure que la première aiguille/le premier trocart se déplace dans une direction d'insertion.

6. Dispositif de perfusion selon la revendication 5, dans lequel :
la première aiguille/le premier trocart est situé(e) à l'intérieur de la première canule de sorte que la première canule reste insérée lorsque la première aiguille/le premier trocart est rétracté(e).

7. Dispositif de perfusion selon la revendication 6, dans lequel :
le mécanisme d'insertion d'outil d'accès transcutané comprend
un premier élément coulissant configuré pour déplacer la première aiguille/le premier trocart dans une direction d'insertion et dans une direction de rétraction ;
un second élément coulissant configuré pour déplacer la première canule dans la direction d'insertion ;
un ressort de torsion ; et
des liaisons couplées entre le ressort de torsion et le premier élément coulissant de sorte que l'énergie stockée dans le ressort de torsion amène les liaisons à déplacer le premier élément coulissant dans la direction d'insertion et la direction de rétraction.

8. Dispositif de perfusion selon la revendication 7, dans lequel :
le mécanisme d'insertion d'outil d'accès transcutané comprend en outre
un cadre recevant de manière coulissante les premier et second éléments coulissants et configuré pour verrouiller les premier et second éléments coulissants dans une position de pré-déploiement et pour verrouiller le second élément coulissant dans une position de post-déploiement.

9. Dispositif de perfusion selon la revendication 8, dans lequel :
le cadre inclut un doigt de came configuré pour verrouiller les premier et second éléments coulissants dans la position de pré-déploiement.

10. Dispositif de perfusion selon la revendication 9, dans lequel :
le doigt de came est configuré pour verrouiller le second élément coulissant dans la position de post-déploiement.

11. Dispositif de perfusion selon la revendication 9, dans lequel :
le mécanisme d'insertion d'outil d'accès transcutané comprend en outre
une barre de libération configurée pour maintenir le doigt de came lorsque le doigt de came verrouille les premier et second éléments coulissants dans la position de pré-déploiement et configurée pour libérer le doigt de came pour permettre aux premier et second éléments coulissants de se déplacer dans la direction d'insertion.

12. Dispositif de perfusion selon la revendication 11, dans lequel :
le mécanisme d'entraînement vient en prise avec la barre de libération et déclenche un mouvement de la barre de libération pour libérer le doigt de came.

13. Dispositif de perfusion selon la revendication 1, dans lequel :
le mécanisme d'entraînement comprend
un piston reçu dans le réservoir ;
un ensemble allongé comprenant un premier élément allongé et un second élément allongé ;
le premier élément allongé s'étendant depuis le piston ;
le second élément allongé étant couplé au premier élément allongé ;
une roue d'entraînement ; et
un mécanisme d'embrayage couplé à la roue d'entraînement, dans lequel le mécanisme d'embrayage est configuré pour permettre le passage du second élément allongé lorsqu'il n'est pas en prise et est configuré pour saisir le second élément allongé lorsqu'il est en prise de sorte que la roue d'entraînement fasse tourner le second élément allongé pour faire avancer le premier élément allongé et le piston dans le réservoir.

14. Dispositif de perfusion selon la revendication 13, dans lequel :
le premier élément allongé comprend un premier élément fileté ;
le second élément allongé comprend un second élément fileté ;
le second élément fileté est en prise par filetage avec le premier élément fileté ; et
le mécanisme d'embrayage est configuré pour permettre le passage du second élément fileté lorsqu'il est n'est pas en prise et est configuré pour saisir le second élément fileté lorsqu'il est en prise de sorte que la roue d'entraînement fasse tourner le second élément fileté pour faire avancer le premier élément fileté et le piston dans le réservoir.

15. Dispositif de perfusion selon la revendication 14, dans lequel :
le premier élément fileté allongé comprend un premier arbre fileté ayant des filetages externes.

16. Dispositif de perfusion selon la revendication 14, dans lequel :
le second élément fileté allongé comprend un second arbre fileté ayant des filetages internes.

17. Dispositif de perfusion selon la revendication 13, dans lequel :
le mécanisme d'embrayage inclut un ressort d'embrayage qui saisit le second élément allongé lorsqu'il est libéré.

18. Dispositif de perfusion selon la revendication 17, dans lequel :
le mécanisme d'embrayage inclut en outre un loquet à ressort configuré pour maintenir le ressort d'embrayage dans une position dans laquelle il n'est pas en prise et configuré pour libérer le ressort d'embrayage de sorte que le ressort d'embrayage se déplace vers une position en prise.

19. Dispositif de perfusion selon la revendication 18, dans lequel :
le loquet à ressort est configuré pour libérer le ressort d'embrayage en réponse à un mouvement de la roue d'entraînement.
